# NEUE EUROPÄISCHE PATENTSCHRIFT

(11) **EP 1 444 214 B2**
(45) Veröffentlichungstag und Bekanntmachung des Hinweises auf die Entscheidung über den Einspruch: **16.12.2009**
(45) Hinweis auf die Patenterteilung: 13.07.2005
(21) Anmeldenummer: 02787377.7
(22) Anmeldetag: 14.11.2002
(51) Int. Cl.: C07D 251/62

(54) **VERFAHREN ZUR HERSTELLUNG VON MELEMFREIEM MELAMIN UND QUENCHER**
METHOD FOR PRODUCING MELEM-FREE MELAMINE AND QUENCHING AGENTS
PROCEDE DE FABRICATION DE MELAMINE EXEMPTE DE MELEM ET REFROIDISSEUR

(30) Priorität: 16.11.2001 AT 18072001; 25.06.2002 DE 10229100
(43) Veröffentlichungstag der Anmeldung: 11.08.2004
(73) Patentinhaber: AMI Agrolinz Melamine International GmbH, 4021 Linz (AT)
(72) Erfinder: SCHRÖDER, Frank, 04683 Albrechtshain (DE); RUECH, Wolfgang, A-4753 Taiskirchen (AT); NEUMÜLLER, Christoph, A-4482 Ennsdorf bei Enns (AT); KOGLGRUBER, Ferdinand, A-4040 Linz (AT); WAGNER, Hans, Christian, A-1235 Wien (AT)
(74) Vertreter: Gross, Felix
(86) Internationale Anmeldenummer: PCT/DE2002/004251
(87) Internationale Veröffentlichungsnummer: WO 2003/045927

(56) Entgegenhaltungen:
- AT-B- 404 018
- US-A- 3 207 744

## Beschreibung

Die Anmeldung betrifft ein Verfahren zur Herstellung von melemfreiem Melamin durch direkte Überführung einer aus einem Hochdruckverfahren erhaltenen Melaminschmelze in eine wässrige alkalische Melaminlösung durch Quenchen mit wässriger alkalihältiger Lösung und anschließender Kristallisation des Melamins. Die Erfindung betrifft auch Quencher mit den Merkmalen des Anspruchs 8.

Bei den Hochdruckverfahren zur Herstellung von Melamin werden im allgemeinen Harnstoffschmelze und gegebenenfalls gasförmiges Ammoniak ohne Anwesenheit eines Katalysators etwa bei Temperaturen zwischen 325 und 450 °C, bevorzugt zwischen 350 und 425 °C und Drücken zwischen 50 und 250 bar zu flüssigem Melamin und Offgas, hauptsächlich bestehend aus Ammoniak und Kohlendioxid, mit geringen Mengen an gasförmigem Melamin, umgesetzt. Diese Reaktionsmischung wird anschließend, je nach Verfahren in unterschiedlicher Weise, unter hohem Druck und hoher Temperatur aufgearbeitet. Im Anschluß daran steht die Melarninverfestigung, welche auf zwei Arten durchgeführt werden kann: Bei den Trockenverfahren wird die Melaminschmelze in Abwesenheit von Wasser mit oder ohne Ammoniak unter gleichzeitigem Entspannen verfestigt, gekühlt und anschließend isoliert. Der Nachteil dieser Trockenverfahren ist jedoch, dass während der Expansion viele Nebenprodukte wie beispielsweise Melam und Melem gebildet werden, welche in den weiteren Verarbeitungsschritten des Melamins, vor allem bei der Verarbeitung zu Melaminharzen und deren Folgeprodukten, stören. Besonders störend ist das Kondensationsnebenprodukt Melem.
In einem kombinierten Trocken- / Nassverfahren nach US 3637686 wird die Melaminschmelze mit Ammoniak in Abwesenheit von Wasser zuerst verfestigt und das feste Melamin anschließend in wässriger ammoniakhältiger Lösung aufgearbeitet, um die im Melamin enthaltenen, teilweise während der Verfestigung gebildeten Nebenprodukte zu entfernen. Bei einem solchen Umkristallisationsschritt kann jedoch die parallel zum Nebenproduktabbau stattfindende Melaminhydrolyse einen beträchtlichen Ausbeuteverlust bedeuten.

Eine andere Möglichkeit, Melamin aus einem Hochdruckprozeß zu verfestigen ist in US 3132143 oder WO 01/36397 beschrieben. Dort wird die aus dem Melaminreaktor direkt erhaltene Reaktionsmischung in einen Quencher entspannt und in Gegenwart von wässrigem Ammoniak und Kohlendioxid in eine kohlendioxid- und nebenprodukthältige Melaminlösung überführt. Der Nachteil des beschriebenen Verfahrens besteht darin, dass beim hydrolytischen Nebenproduktabbau in Gegenwart von Kohlendioxid aufgrund des relativ niedrigen pH-Wertes sehr hohe Temperaturen und lange Verweilzeiten benötigt werden, um den gewünschten Abbaugrad der Nebenprodukte zu erreichen.
Eine weitere Möglichkeit, die Melaminschmelze aus einem Hochdruckprozeß zu verfestigen, ist in WO 00/29393 beschrieben. Dort wird die Melaminschmelze mit Hilfe einer wässrigen ammoniakhältigen Lösung in eine Melaminsuspension überführt und daraus das Melamin isoliert. Der Nachteil bei diesem Verfahren besteht darin, dass wie in den Trockenverfahren während des Verfestigungsschrittes zusätzlich Nebenprodukte gebildet werden, welche aus der Melaminsuspension nur durch sehr lange Verweilzeiten in der wässrigen Phase entfernt werden können, was hohen Melaminverlust durch Hydrolyse bedeutet. Darüber hinaus ist bei Suspensionsfahrweise die Gefahr von Ablagerungen und Verstopfungen durch auskristallisierende Nebenprodukte nachteilig.

Es stellte sich demnach die Aufgabe, ein Verfahren zu finden, mit dem es möglich ist, bei einer größtmöglichen Ausbeute des Melamins, einen Melemgehalt von unter 50 ppm zu erreichen. Unerwarteterweise konnte die Aufgabe dadurch gelöst werden, dass die aus einem Hochdruckverfahren kommende Melaminschmelze nach Abtrennen der Offgase mit wässriger alkalischer NaOH- oder KOH-Lösung gequencht, dabei in eine wäßrige alkalische Melaminlösung überführt und durch Kristallisation festes Melamin erhalten wird, wobei aus der Melaminschmelze vor dem Quenchen die sauerstoffhältigen Nebenprodukte weitgehend und die Kondensationsnebenprodukte teilweise entfernt werden.

Gegenstand der vorliegenden Erfindung ist demnach ein Verfahren nach Anspruch 1.

Durch erfindungsgemäße Überführung der Melaminschmelze in eine Melaminlösung ist es zum einen möglich, die im Hochdruckteil der Melaminanlage gebildete Melaminqualität ohne zusätzliche Nebenproduktbildung in den wässrigen Aufarbeitungsteil zu überführen. Zum anderen ist es durch Verwendung einer alkalihältigen Lösung zum Quenchen der Melaminschmelze möglich, die mit der Melaminschmelze in den wässrigen Aufarbeitungsteil eingebrachten Nebenprodukte bereits im Quencher abzubauen, wodurch sich die gesamte Verweilzeit des Melamins im wässrigen Aufarbeitungsteil und somit der Melaminverlust durch Hydrolyse minimieren lässt.

Die nach dem vorliegenden Verfahren aufzuarbeitende Melaminschmelze stammt aus einem Hochdruckverfahren und wird mit einer Temperatur von etwa 330 bis 400 °C, bevorzugt von etwa 330 bis 380 °C, besonders bevorzugt von etwa 330 bis 360 °C und einem Druck von etwa 50 bis 600 bar, bevorzugt von etwa 50 bis 250 bar, besonders bevorzugt von etwa 70 bis 170 bar dem Quencher zugeführt.
In der Melaminschmelze aus einem Hochdruckverfahren sind zwei Arten von Nebenprodukten enthalten. Zu den sauerstoffhältigen Nebenprodukten in der Melaminschmelze gehören beispielsweise Kohlendioxid, Ammelin, Ammelid, Ureidomelamin oder nicht umgesetzter Harnstoff. Als Kondensationsnebenprodukte bezeichnet man beispielsweise Melem oder Melam.
Im vorliegenden Verfahren kann jede aus einem Hochdruckverfahren stammende Melaminschmelze, wobei aus der Melaminschmelze vor dem Quenchen die sauerstoffhältigen Nebenprodukte weitgehend und die Kondensationsprodukte teilweise entfernt werden, nach Abtrennung der Reaktionsoffgase verwendet werden. Besonders reines Melamin kann nach dem vorliegenden Verfahren erhalten werden, wenn die Melaminschmelze im Hochdruckteil der Melaminanlage vorgereinigt wird. Dabei ist es besonders vorteilhaft, die sauerstoffhältigen Nebenprodukte beispielsweise durch Behandeln der Melaminschmelze mit gasförmigem Ammoniak weitgehend und die Kondensationsnebenprodukte beispielsweise durch Kühlen und / oder Verweilen der Melaminschmelze unter hohem Ammoniakdruck teilweise aus der Melaminschmelze zu entfernen. Besonders vorteilhaft ist es weiters, im vorliegenden Verfahren ammoniakgesättigte Melaminschmelze zu verwenden.

Die Eintragung der Melaminschmelze in den Quencher erfolgt bevorzugt durch Einsprühen der Melaminschmelze, um eine feine Verteilung derselben zu erreichen. Gleichzeitig mit der Melaminschmelze wird eine wässrige alkalihältige Lösung bevorzugt in Sprühkegelform in den Quencher eingebracht. Die Einsprühvorrichtungen sind bevorzugt so angeordnet, dass der Kontakt zwischen Melaminschmelze und wässriger alkalihältiger Lösung unmittelbar nach dem Eintreten der Melaminschmelze in den Apparat erfolgt. Beispielsweise erfolgt der Eintritt der Melaminschmelze mittig im oberen Apparateteil, während die wässrige alkalihältige Lösung von oben über eine oder mehrere Düsen in den Quencher eingesprüht wird. Eine andere Möglichkeit der Einbringung der Melaminschmelze und der wässrigen alkalihältigen Lösung ist die Verwendung einer Zweistoffdüse. Auf diese Weise, wird die Melaminschmelze beim Kontakt mit der eingesprühten Lösung direkt in die gelöste Form überführt. Es ist auch möglich, die Gesamtmenge der zum Quenchen der Melaminschmelze eingebrachten wässrigen alkalihältigen Lösung auf zwei oder mehrere Teilmengen, beispielsweise im Mengenverhältnis von etwa 1:1 bis 1,5:1 aufzuteilen, von denen eine Teilmenge von oben in den Apparat eingebracht wird, während die zweite Teilmenge zur besseren Durchmischung seitlich im unteren Apparateteil eingeleitet wird.
Die Menge der in den Quencher eingetragenen wässrigen alkalihältigen Lösung ist beispielsweise so, dass die aus dem Quencher ausgetragene wässrige alkalische Melaminlösung eine Konzentration von etwa 5 bis 10 Gew%, bevorzugt von etwa 6 bis 9 Gew% Melamin aufweist.
Die Temperatur der in den Quencher eingebrachten wässrigen alkalihältigen Lösung wird so gewählt, dass das Quenchen der Melaminschmelze bei einer Temperatur von etwa 100 bis 150°C, bevorzugt von etwa 110 bis 145 °C, besonders bevorzugt von etwa 120 bis 140 °C erfolgt.
Der Druck beim Quenchen beträgt 1 bis 7 bar, bevorzugt, 2 bis 6 bar, besonders bevorzugt 3 bis 5 bar.

Der Alkaligehalt der in den Quencher eingespeisten wässrigen alkalihältigen Lösung beträgt etwa 0,05 bis 0,5 Gew%, bevorzugt etwa 0,05 bis 0,3 Gew%. Als alkalischer Bestandteil der Lösung wird Natriumhydroxid, oder Kaliumhydroxid verwendet, bevorzugt wird Natriumhydroxid eingesetzt. Bevorzugt stammt der alkalische Bestandteil in der zum Quenchen verwendeten wässrigen alkalihältigen Lösung vorwiegend aus der Mutterlauge der Melaminkristallisation.
Die zum Quenchen verwendete Lösung enthält demnach bevorzugt einen rückgeführten Teil der Kristallisationsmutterlaugen, darüber hinaus können die kondensierten schwach ammoniakhältigen Brüden der Melaminkristallisation enthalten sein, weiters ist in der zum Quenchen verwendeten Lösung Frischkondensat enthalten.

Die Betriebsweise des Quenchers entsprechend der vorliegenden Erfindung bietet mehrere Vorteile, welche im folgenden erläutert werden.
Durch die erfindungsgemäße Überführung der Melaminschmelze in eine Melaminlösung im Quencher ist es möglich, den Übergang von der Schmelzephase in die wässrige Aufarbeitung der Melaminanlage ohne Qualitätsverschlechterung durch Nebenproduktbildung zu gewährleisten. Das bedeutet, dass im wässrigen Aufarbeitungsteil ausschließlich die mit der Melaminschmelze bereits eingetragenen Nebenprodukte abgebaut werden müssen, jedoch im Unterschied zu den reinen Trockenverfahren oder zu den Verfahren, die in Suspension arbeiten, keine zusätzlich während der Entspannung gebildeten Nebenprodukte.
Die Verweilzeit im wässrigen Aufarbeitungsteil wird durch den im Quencher vorhandenen Nebenproduktgehalt definiert, das heißt, je geringer der Nebenproduktgehalt, desto kürzer kann die Verweilzeit gewählt werden, um die jeweils geforderte niedrige Nebenproduktkonzentration im Endprodukt zu erreichen.
Eine möglichst geringe Verweilzeit ist wünschenswert, um die gleichzeitig mit dem Nebenproduktabbau stattfindende Melaminhydrolyse möglichst gering zu halten.
Um die gemeinsam mit dem Melamin in die wässrige Aufarbeitung eingetragenen Nebenprodukte durch Hydrolyse abbauen zu können, müssen die Nebenprodukte in gelöster Form vorliegen. Da die Nebenprodukte Melam und Melem schwerer löslich sind als Melamin, liegen diese bei Suspensionsfahrweise im Quencher in großteils fester Form vor, während das leichter lösliche Melamin auch bei Suspensionsfahrweise in der mit der Suspension im Gleichgewicht stehenden Lösung in gelöster Form vorhanden ist. Dies bedeutet, dass bei Suspensionsfahrweise im Quencher zwar die unerwünschte Melaminhydrolyse stattfindet, der gewünschte Nebenproduktabbau kann jedoch nicht stattfinden.
Im Gegensatz dazu kann bei einem Verfahren entsprechend der vorliegenden Erfindung bereits im Quencher der hydrolytische Nebenproduktabbau beginnen, da bereits im Quencher alle im Melamin vorhandenen Nebenprodukte in Lösung sind.

Der hydrolytische Nebenproduktabbau geht umso rascher vor sich, je höher der pH-Wert in der Lösung ist. Wird der Nebenproduktabbau bei geringen pH-Werten durchgeführt, ist es nötig, die Reaktion bei entsprechend hohen Temperaturen, im allgemeinen bei Temperaturen von mehr als 150 °C und mit langen Verweilzeiten durchzuführen. Dies ist jedoch aufgrund der gleichzeitig ablaufenden Melaminhydrolyse nicht wünschenswert. Durch die entsprechend der vorliegenden Erfindung im Quencher vorhandene freie Natronlauge und den daraus folgenden hohen pH-Wert von etwa pH = 9 bis pH = 12 ist es möglich, die Temperatur während des Nebenproduktabbaus niedrig zu halten und dennoch eine ausreichend rasche Reaktion zu erreichen.

Die im Quencher erhaltene Melaminlösung wird gegebenenfalls nach Versetzen mit mit konzentrierter wässriger alkalihältiger Lösung gegebenenfalls verweilen gelassen. Die Verweilzeit beträgt etwa 5 bis 60 min, bevorzugt etwa 20 bis 40 min. Die erhaltene wässrige alkalische Melaminlösung wird, gegebenenfalls nach Entfernung von Ammoniak, Verdünnen, Behandlung mit Aktivkohle und Einstellen des pH-Wertes einer Kristallisationseinheit zugeführt, wo beispielsweise durch Temperaturerniedrigung und / oder Anlegen eines Vakuums das Melamin auskristallisiert wird. Anschließend wird das Melamin filtriert, getrocknet und isoliert.

Mit dem vorliegenden Verfahren ist es möglich, Melamin mit einem Melamgehalt von < 1000 ppm und einem Melemgehalt von < 50 ppm herzustellen, welches jeder gewünschten Weiterverarbeitung zugeführt werden kann.

Die im Krisallisator abgezogene alkalische Mutterlauge kann, gegebenenfalls nach Vermischen mit dem Filtrat der Melaminfiltration, auf zwei Teilströme im Verhältnis von etwa 2,5 : 1 bis 3,0 : 1 aufgeteilt werden. Der erste Teilstrom wird in die wässrige Melaminaufarbeitung rückgeführt. Dabei ist es möglich, einen Teil oder die gesamte rückgeführte Mutterlauge zum Quenchen der Melaminschmelze zu verwenden. Der zweite Teilstrom aus Kristallisator-Mutterlauge und Filtrat wird ausgeschleust und einer Abwasseraufarbeitung zugeführt.

### Beispiel:

Melaminschmelze aus einer Hochdruck-Melaminanlage wurde mit einer Temperatur von 360 °C und einem Druck von 245 bar in einen Quencher, welcher bei einem Druck von 2,5 bar betrieben wurde, eingesprüht. Die Melaminschmelze enthielt eine Ausgangskonzentration von 11.850 ppm Melam sowie 670 ppm Melem, war weitgehend frei von sauerstoffhältigen Verunreinigungen sowie annähernd ammoniakgesättigt. Gleichzeitig mit der Melaminschmelze wurde eine wässrige Lösung mit einem NaOH-Gehalt von 0,3 Gew% in den Quencher eingebracht. Die Mengenverhältnisse von Melaminschmelze und wässriger NaOH-Lösung wurden so gewählt, dass im Quencher eine 5% ige Lösung an Melamin und Nebenprodukten vorlag. Die Temperatur der Melaminlösung im Quencher betrug 130 °C. Nach einer Verweilzeit von 30 min unter diesen Temperatur- und Druckbedingungen wurde die Lösung abgekühlt, die erhaltene Melaminsuspension neutralisiert, getrocknet und das Melamin analysiert. Der Melamgehalt im Melamin betrug 810 ppm, der Melemgehalt < 50 ppm.

### Vergleichsbeispiel:

Die Versuchsdurchführung war analog zu Beispiel 1. Die in den Quencher eingetragene Melaminschmelze war weitgehend frei von sauerstoffhältigen Nebenprodukten, die Kondensationsnebenprodukte Melam und Melem wurden jedoch durch Vorreinigung im Hochdruckteil nicht entfernt. Somit betrug der Ausgangsgehalt der Melaminschmelze an Melam 35.000 ppm, der Melemgehalt war 5.600 ppm. Die Melaminschmelze wurde mit 380 °C und 70 bar in den Quencher, der bei 2,5 bar betrieben wurde, eingetragen. Gleichzeitig mit der Melaminschmelze wurde eine wässrige Lösung mit einem NaOH-Gehalt von 0,2 Gew% in den Quencher eingebracht. Die Mengenverhältnisse von Melaminschmelze und wässriger NaOH-Lösung wurden so gewählt, dass im Quencher eine 8% ige Lösung an Melamin und Nebenprodukten vorlag. Die Temperatur der Melaminlösung im Quencher betrug 130 °C. Nach einer Verweilzeit von 60 min im Quencher wurde die Lösung abgekühlt, die erhaltene Melaminsuspension neutralisiert, getrocknet und das Melamin analysiert. Der Melamgehalt im Melamin betrug 2.700 ppm, der Melemgehalt 100 ppm.

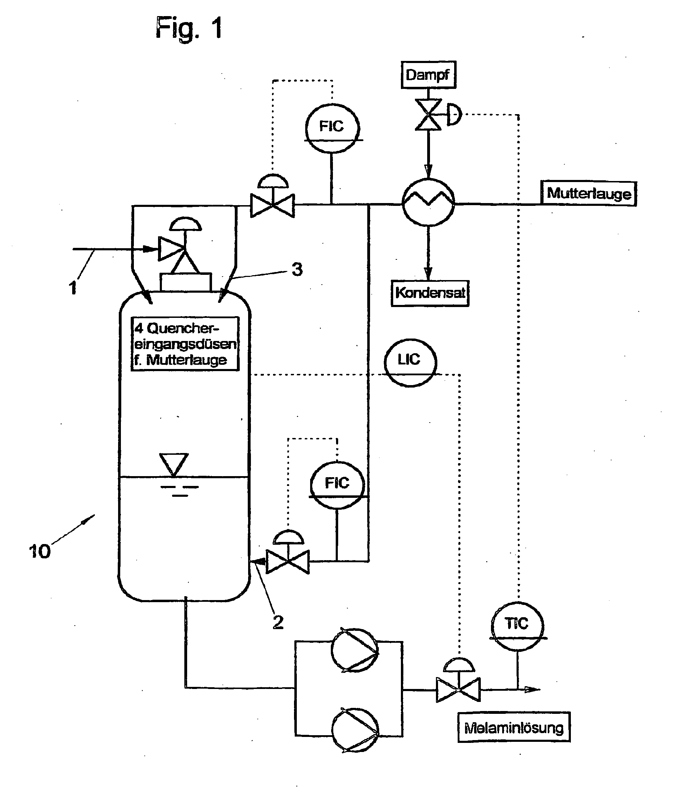

In Fig. 1 wird ein Quncher 10 dargestellt, der für das erfindungsgemäße Verfahren eingesetzt wird.

Oben am Quencher 10 ist ein Melaminschmelzeeingang 1 angeordnet, wobei seitlich davon vier Quenschereinlassdüsen für heiße Mutterlauge (wässrige alkalihaltige Lösung) angeordnet sind. Die ca. 120°C heiße Mutterlauge vermischt sich mit der ca. 350 °C heißen Melaminschmelze, so dass sich im oberen Bereich des Quenschers eine Mischungstemperautr von ca. 140 °C einstellt Diese Muterlauge- Melamin Mischung wird anschließend erneut mit ca. 120°C kühler Mutterlauge vermischt, so dass sich unten im Quencher 10 eine Temperatur von ca. 130°C einstellt. Die Einspeisung 2 im unteren Bereich erfolgt tangential zur Wandung des Behälters.

Dies hat folgende Vorteile, dass im oberen Apparatebereich eine höhere Temperatur, durch Zumischen von kälteterem Mutterlauge-Strom im unteren Apparatebereich erzielt eine Abkühlung erreicht wird.

Durch die höhere Temperatur im oberen Bereich des Quenchers stellt sich oben auch ein höherer Druck als im unteren Apparatebereich ein (= Sattdampfdruck entsprechend der Temperatur). Durch Zumischen von kühlerer Flüssigkeit im unteren Bereich erniedrigt sich auch der Druck.

Dies ist positiv, da dadurch der NPSH-Wert (Haltedruckhöhe) der Sumpfabzugspumpe vergrößert, womit sich die Gefahr der Pumpenkavitation verringert.

Eine bessere Durchmischung wrd dadurch ereicht, dass der untere Mutterlaugenstrom tangential einströmt

Am Kopf des Quenchers befinden sich die vier Einsprühdüsen für die Mutterlauge. Jede der Düsen ist ausgelegt auf einen bestimmten zu verarbeitenden Mengenstrom. Nur bei diesem Strom arbeiten die Düsen (hinsichtlich der Sprühgeometrie u. Verstopfungsrisiko) optimal.

Vorteil der durch die zweigeteilte Zufuhr hinsichtlich des optimalen Betriebspunktes der Düsen erzielt wird: Auch bei Teillast der Melaminanlage d.h. Zufuhr von < 100% der Melaminschmelzemenge in den Quencher können die Mutterlauge-Einsprühdüsen immer mit demselben (nämlich optimalen) Mengenstrom beaufschlagt werden, indem z.B. die untere Mutterlaugezufuhr au f »Null » gestellt wird. Dadurch wird auch bei Teillast der Melaminanlage immer eine optimale Verteilung / optimales Düsen-Sprühbild der Mutterlauge im Quencher erreicht.

## Patentansprüche

1. Verfahren zur Herstellung von melemfreiem Melamin durch wässrige Aufarbeitung einer aus einem Hochdruckverfahren erhaltenen Melaminschmelze, **dadurch gekennzeichnet, dass** die Melaminschmelze nach Abtrennen der Offgase mit einer wässrigen alkalischen NaOH- oder KOH-Lösung gequencht, dabei in eine wässrige alkalische Melaminlösung überführt und durch Kristallisation festes Melamin erhalten wird, wobei aus der Melaminschmelze vor dem Quenchen die sauerstoffhältigen Nebenprodukte weitgehend und die Kondensationsnebenprodukte teilweise entfernt wurde.

2. Verfahren nach Anspruch 1, **dadurch gekennzeichnet, dass** die wässrige alkalihältige Lösung eine aus der Melaminkristallisation rückgeführte Mutterlauge enthält.

3. Verfahren nach mindestens einem der vorhergehenden Ansprüche, **dadurch gekennzeichnet, dass** das Quenchen der Melaminschmelze mit wässriger alkalihältiger Lösung bei einer Temperatur von 100 bis 150 °C erfolgt.

4. Verfahren nach mindestens einem der vorhergehenden Ansprüche, **dadurch gekennzeichnet, dass** das Quenchen der Melaminschmelze mit wässriger alkalihältiger Lösung bei einem Druck von 1 bis 7 bar erfolgt.

5. Verfahren nach mindestens einem der vorhergehenden Ansprüche, **dadurch gekennzeichnet, dass** die Konzentration an Alkali in der wässrigen alkalihältigen Lösung zwischen 0,05 und 0,5 Gew% beträgt.

6. Verfahren nach mindestens einem der vorhergehenden Ansprüche, **dadurch gekennzeichnet, dass** die wässrige alkalische Melaminlösung, gegebenenfalls nach Versetzen mit konzentrierter wässriger alkalihältiger Lösung für 5 bis 60, bevorzugt 20 bis 40 min verweilen gelassen wird.

7. Verfahren nach mindestens einem der vorhergehenden Ansprüche, **dadurch gekennzeichnet, dass** die wässrige alkalische Melaminlösung, gegebenenfalls nach Versetzen mit konzentrierter wässriger alkalihältiger Lösung für 20 bis 40 min verweilen gelassen wird.

8. Quencher zur Verwendung in einem Verfahren nach Anspruch 1, **dadurch gekennzeichnet, dass** wässrige alkalihaltige Lösung an zwei räumlich getrennten Zuläufen (2, 3) zugegeben wird und wobei mindestens ein Zulauf im Wesentlichen so im unteren Bereich des Quenchers angeordnet ist, dass das einströmende Fluid tangential zur Wandung des Quenchers gerichtet ist, und die räumlich getrennten Zuläufe (2, 3) außerhalb des Quenchers miteinander verbunden sind, und wobei eine Aufteilung des Stromes der wässrigen alkalischen Lösung vor Zuleitung zu den räumlich getrennten Zuläufen (2, 3) erfolgt.

9. Quencher nach Anspruch 8 **dadurch gekennzeichnet, dass** ein Zulauf (3) im Bereich der Zufuhr der heißen Melaminschmelze angeordnet ist.

10. Quencher nach Anspruch 8 oder 9, **dadurch gekennzeichnet, dass** ein Zulauf (2) im Bereich der Zufuhr kühler Mutterlauge angeordnet ist.

## Claims

1. Process for preparing melem-free melamine by aqueous work-up of a melamine melt obtained from a high pressure process, **characterized in that** the melamine melt, after separating off the off-gases, is quenched with an aqueous alkali NaOH- or KOH-solution, converted in the process into an aqueous alkaline melamine solution and then solid melamine is obtained by crystallization, whereby the oxygen-containing by-products have been substantially removed, and the condensation by-products removed in part, from the melamine melt before quenching.

2. Process according to claim 1, **characterized in that** the aqueous alkali-containing solution comprises a mother liquor re-circulated from the melamine crystallization.

3. Process according to at least one of the preceding claims, **characterized in that** the melamine melt is quenched with an aqueous alkali-containing solution at a temperature of from 100 to 150°C.

4. Process according to at least one of the preceding claims, **characterized in that** the melamine melt is quenched with an aqueous alkali-containing solution at a pressure of from 1 to 7 bar.

5. Process according to at least one of the preceding claims, **characterized in that** the concentration of alkali in the aqueous alkali-containing solution is between 0.05 and 0.5 % by weight.

6. Process according to at least one of the preceding claims, **characterized in that** the aqueous alkaline melamine solution, if appropriate after addition of concentrated aqueous alkali-containing solution, is allowed to stand from 5 to 60 min, preferably from 20 to 40 min.

7. Process according to at least one of the preceding claims, **characterized in that** the aqueous alkaline melamine solution, if appropriate after addition of concentrated aqueous alkali-containing solution, is allowed to stand for from 20 to 40 min.

8. Quencher for use in a process according to claim 1, **characterized in that** aqueous alkali-containing solution is added at two spatially separated infeeds (2, 3), and whereby at least one infeed is disposed essentially in the lower area of the quencher such that the influent liquid is orientated tangentially to the wall of the quencher, and the spatially separated infeeds (2, 3) are connected to each other outside of the quencher, and whereby a division of the stream of the aqueous alkali-solution occurs before supply to the spatially separated infeeds (2, 3).

9. Quencher according to claim 8, **characterized in that** that an infeed (3) is disposed in the region of the feed of the hot melamine melt.

10. Quencher according to claim 8 or 9, **characterized in that** an infeed (2) is disposed in the region of the feed of cool mother liquor.

## Revendications

1. Procédé en vue de la fabrication de mélamine exempte de melem, par traitement aqueux d'une masse en fusion de mélamine, obtenue à partir d'un procédé haute pression, **caractérisé en ce que** la masse en fusion de mélamine, après la séparation des gaz de rejet, subit une trempe à l'aide d'une solution aqueuse alcaline de NaOH ou KOH, **en ce que** cette dernière est convertie, de ce fait, en une solution de mélamine alcaline aqueuse et **en ce que** l'on obtient, par cristallisation, de la mélamine solide, dans lequel on élimine, hors de la masse en fusion de mélamine, avant la trempe, dans une large mesure les produits secondaires contenant de l'oxygène et partiellement les produits secondaires de condensation.

2. Procédé selon la revendication 1, **caractérisé en ce que** la solution aqueuse contenant des alcalins contient une solution-mère recyclée à partir de la cristallisation de la mélamine.

3. Procédé selon au moins l'une quelconque des revendications précédentes, **caractérisé en ce que** la trempe de la masse en fusion de mélamine se fait à l'aide d'une solution aqueuse contenant des alcalins, à une température de 100 à 150 °C.

4. Procédé selon au moins l'une quelconque des revendications précédentes, **caractérisé en ce que** la trempe de la masse en fusion de mélamine se fait à l'aide d'une solution aqueuse contenant des alcalins, à une pression de 1 à 7 bars.

5. Procédé selon au moins l'une quelconque des revendications précédentes, **caractérisé en ce que** la concentration en alcalins dans la solution aqueuse contenant des alcalins est comprise entre 0,05 et 0,5% en poids.

6. Procédé selon au moins l'une quelconque des revendications précédentes, **caractérisé en ce que** la solution de mélamine alcaline aqueuse est laissée au repos pendant une période de 5 à 60 minutes, de préférence de 20 à 40 minutes, le cas échéant, après mélange à une solution concentrée aqueuse contenant des alcalins.

7. Procédé selon au moins l'une quelconque des revendications précédentes, **caractérisé en ce que** la solution de mélamine alcaline aqueuse est laissée au repos pendant une période de 20 à 40 minutes, le cas échéant, après mélange à une solution concentrée aqueuse contenant des alcalins.

8. Dispositif de trempe en vue de l'utilisation dans un procédé selon la revendication 1, **caractérisé en ce que** de la solution aqueuse contenant des alcalins est ajoutée dans deux admissions (2, 3) séparées dans l'espace et dans lequel au moins une admission est pour l'essentiel disposée dans le domaine inférieur du dispositif de trempe de telle sorte que le fluide affluant soit dirigé d'une manière tangentielle par rapport à la paroi du dispositif de trempe, et les admissions (2, 3) séparées dans l'espace sont raccordées l'une à l'autre à l'extérieur du dispositif de trempe, et dans lequel on répartit le courant de la solution aqueuse contenant des alcalins avant de l'amener aux admissions (2, 3) séparées dans l'espace.

9. Dispositif de trempe selon la revendication 8, **caractérisé en ce qu'**une admission (3) est disposée dans le domaine de l'alimentation de la masse en fusion chaude de mélamine.

10. Dispositif de trempe selon la revendication 8 ou 9, **caractérisé en ce qu'**une admission (2) est disposée dans le domaine de l'alimentation de la solution-mère refroidie.
